# EUROPEAN PATENT APPLICATION

(11) **EP 4 512 897 A1**
(43) Date of publication of application: **26.02.2025**
(21) Application number: 22938081.1
(22) Date of filing: 31.05.2022
(51) Int. Cl.: C12N 15/11, A61P 9/00, A61P 9/04, A61P 9/06

(54) **MUTANT OF SMALL MOLECULAR CHAPERONE PROTEIN MOG1-ENCODING GENE AND USE THEREOF**

(30) Priority: 21.04.2022 CN 202210425547
(71) Applicant: Huazhong University of Science and Technology, Wuhan, Hubei 430074 (CN)
(72) Inventor: WANG, Qing, Wuhan, Hubei 430074 (CN); XIONG, Hongbo, Wuhan, Hubei 430074 (CN)
(74) Representative: Straus, Alexander
(86) International application number: PCT/CN2022/096227
(87) International publication number: WO 2023/201851

(57) **Abstract**

Provided are a mutant of a small molecular chaperone protein MOG1-encoding gene and use thereof. The mutant is more effective than a wild-type MOG1 in enhancing myocardial sodium current, and the mutant of the small molecular chaperone protein MOG1-encoding gene and a mutant of the small molecular chaperone protein MOG1 are used in the preparation of medicaments for the treatment of diseases such as Brugada syndrome, arrhythmia, dilated cardiomyopathy, or heart failure.

## Description

### Technical Field

The invention belongs to the technical field of biomedicine, and more specifically, relates to a mutant of a small molecule chaperone protein MOG1-encoding gene and an application thereof.

### Background Technology

Cardiac arrhythmias cause millions of sudden deaths each year worldwide. Most antiarrhythmic drugs do not prevent arrhythmias but can induce them. Brugada syndrome (BrS) is a fatal ventricular arrhythmia syndrome characterized by ST-segment elevation in the precordial leads of the electrocardiogram (ECG), ventricular tachycardia (VT) or ventricular fibrillation (VF), leading to syncope, seizures, and sudden death. Brugada syndrome causes a large number of sudden cardiac deaths in relatively young, healthy people aged 30 to 40 years, especially in Southeast Asian populations, in which the syndrome causes 4% of sudden cardiac deaths. However, available treatments for the syndrome are limited. Although implantable cardioverter-defibrillators (ICDs) are commonly used, the devices do not prevent arrhythmias. In addition, ICDs often cause side effects including allergic reactions, infections, and inappropriate discharges. Therefore, it is necessary to discover effective therapies for Brugada syndrome and the associated arrhythmias thereof.

In 1998, we reported the first gene causing Brugada syndrome, SCN5A, which encodes the cardiac sodium channel Nav1.5. The cardiac sodium channel Nav1.5 is responsible for the initiation and conduction of action potentials in human cardiomyocytes. SCN5A mutations causing arrhythmias such as Brugada syndrome are loss-of-function mutations. To date, SCN5A mutations are the most common variants causing Brugada syndrome, accounting for 25% to 30% of BrS cases. In 2008, we identified a small molecule chaperone protein MOG1 of only 20 kDa that can interact with Nav1.5 through yeast two-hybrid screening. Further studies showed that MOG1 facilitated the transfer of Nav1.5 from the endoplasmic reticulum to the cell surface, but did not affect the sodium channel dynamics or the conductance of individual sodium channels. In 2003, we found that in HEK293 cells, the Brugada syndrome mutation p.G1743R caused the cardiac sodium channel Nav1.5 to be unable to be properly transferred to the cell membrane, which significantly reduced the cardiac sodium current, while high expression of MOG1 could restore the defects. In addition, SCNSA mutation p.D1275N causes arrhythmias such as sick sinus syndrome and atrial fibrillation, and dilated cardiomyopathy and heart failure. The SCNSA mutation p.D1275N also caused the cardiac sodium channel Nav1.5 to be unable to be properly transferred to the cell membrane, which significantly reduced the cardiac sodium current, while high expression of MOG1 could restore the defects. We recently established SCNSA mutant p.G1743R knock-in (KI) mice (Scn5aG1746R/+). Homozygous KI mice die before embryonic day E12. However, heterozygous Scn5aG1746R/+ KI mice recapitulated the clinical features of Brugada syndrome, including ST-segment abnormalities (prominent J waves) of the electrocardiogram and spontaneous ventricular tachyarrhythmias (VT), seizures, and sudden death. The potential mechanism of ventricular tachycardia is the shortened cardiac action potential duration, late phase 3 phases early afterdepolarizations (EADs), and sodium current density reduction. We further found that AAV-MOG1 gene therapy using adeno-associated-virus AAV as a vector can increase the expression of exogenous MOG1 gene, increase the expression of cardiac sodium channel Nav1.5 on the cell surface, increase cardiac sodium current, normalizes cardiac action potential abnormalities, eliminates J waves, and blocks arrhythmic symptoms in Scn5aG1746R/+ mice. Humanized KI mice carrying the SCNSA mutation p.D1275N have been reported previously. We found that p.D1275N humanized KI heterozygous mice developed features of arrhythmias, dilated cardiomyopathy, and heart failure. The main causes of the disease are the reduced expression of cardiac sodium channel Nav1.5 on the cell surface and the reduced cardiac sodium current density. AAV-MOG1 gene therapy also increased cell surface Nav1.5 expression, enhanced cardiac sodium current density, and restored the dilated cardiomyopathy and arrhythmia phenotypes in humanized KI heterozygous mice. The studies have shown that AAV-MOG1 gene therapy can treat diseases caused by SCNSA mutations such as Brugada syndrome, ventricular tachycardia, ventricular fibrillation, sick sinus syndrome, atrial fibrillation, dilated cardiomyopathy, and heart failure.

However, we found that only high doses of MOG1 could increase cardiac sodium current by about 1.6 times, which adds certain difficulties to future clinical gene therapy. The main reason is that we need to prepare ultra-high titer AAV-MOG1 virus preparations in order to inject ultra-high doses of the virus into patients. To address this issue, we tried to mutate the MOG1 protein and discovered a super-strong MOG1 mutant. We found that the E50K MOG1 mutant effectively increased cell surface Nav1.5 expression and enhanced cardiac sodium current density by 3.7-fold in HEK293/Nav1.5 cells. Meanwhile, in rat cardiomyocytes, the E50K MOG1 mutant also significantly increased cardiac sodium current density. The main molecular mechanism is: the E50K MOG1 mutant reduces the binding strength with the small molecule GTPase Ran, reduces the amount of MOG1 in the cell nucleus and enriches in the cytoplasm, and participates in the transfer of the cardiac sodium channel Nav1.5 to the cell membrane to a greater extent; at the same time, the protein stability of the E50K MOG1 mutant was also improved. In addition, compared with wild-type MOG1, a low dose of E50K MOG1 mutant can completely restore the reduced cardiac sodium current density caused by SCN5A mutation. Therefore, we believe that MOG1 mutants that affect the interaction with Ran and increase MOG1 protein stability can be more effectively used in AAV-MOG1-based gene therapy.

### SUMMARY OF INVENTION

The invention solves the technical problem in the related art that the wild-type MOG1 requires a high dosage and the effort is poor in increasing cardiac sodium current. The invention uses a mutant of a small molecule chaperone protein MOG1-encoding gene and a mutant of a small molecule chaperone protein MOG1 to prepare a use for treatment of diseases such as Brugada syndrome, arrhythmia, dilated cardiomyopathy, or heart failure. The mutant of the invention is more effective than a wild-type MOG1 in enhancing myocardial sodium current.

According to the first aspect of the invention, a mutant of a small molecule chaperone protein MOG1-encoding gene is provided, and a base sequence of the mutant is shown in SEQ ID NO: 1.

According to the first aspect of the invention, a mutant of a small molecule chaperone protein MOG1 is provided, and an amino acid sequence of the mutant is shown in SEQ ID NO: 2.

According to the first aspect of the invention, a use of the mutant of the small molecule chaperone protein MOG1-encoding gene being used for preparing a drug for treatment of Brugada syndrome, arrhythmia, dilated cardiomyopathy, or heart failure is provided.

Preferably, the arrhythmia is ventricular tachycardia, ventricular fibrillation, sick sinus syndrome, or atrial fibrillation.

Preferably, the mutant of the small molecule chaperone protein MOG1-encoding gene is used to enhance the sodium current density of cardiomyocytes.

According to the first aspect of the invention, a use of the mutant of the small molecule chaperone protein MOG1 being used for preparing a drug for treatment of Brugada syndrome, arrhythmia, dilated cardiomyopathy, or heart failure is provided.

Preferably, the arrhythmia is ventricular tachycardia, ventricular fibrillation, sick sinus syndrome, or atrial fibrillation.

Preferably, the mutant of the small molecule chaperone protein MOG1 is used to enhance the sodium current density of cardiomyocytes.

In general, the technical solution conceived by the invention has the following technical advantages compared with the related art.
(1) The adeno associated virus (AAV) vector used for gene therapy can only package genes smaller than 4.3 kb in size, and the coding region of the SCNSA gene is 6048 bp, which exceeds the packaging capacity of the adeno associated virus (AAV) vector. To overcome this limitation, we packaged the MOG1 gene, which is only 561 bp, into an adeno associated virus (AAV) vector, and a gene therapy technology has been developed to treat diseases such as Brugada syndrome, ventricular tachycardia, ventricular fibrillation, sick sinus syndrome, atrial fibrillation, dilated cardiomyopathy, and heart failure. The gene therapy technology of the invention has reference significance for other genes and diseases that cannot be used for gene therapy because the genes are too large.
(2) Although AAV-MOG1 vectors packaging wild-type MOG1 can be used for related gene therapy, the MOG1 based super-functional mutants developed by the invention (including, for example, mutants destroying MOG1-Ran binding and mutants improving MOG1 stability) are more effective, which reduces the amount of virus injected into patients, and are easier to use in clinical treatment.
(3) The molecular mechanism by which the E50K MOG1 mutant in the invention enhances myocardial sodium current more effectively than wild-type MOG1: In HEK293/Nav1.5 cells, the E50K MOG1 mutant enhances myocardial sodium current more effectively than wild-type MOG1. MOG1 is a small protein of approximately 22 kD originally discovered to be a Ran-GTP release factor regulating the level of Ran-GTP in the cell nucleus and is involved in protein nuclear transfer. MOG1 is mainly localized in the cell nucleus, and the interaction thereof with Ran is highly conserved in evolution. The mouse MOG1 mutant R30A is hyperactive for GTP release by enhancing the binding to Ran-GTP. Recently, nuclear magnetic resonance (NMR)-based structural analysis combined with biochemical characterization revealed that several salt bridges (RanR95-MOG150, RanR106-MOG1D27, RanK130-MOG1D70, RanK132-MOG153, and RanK134-MOG153) are involved in the interaction between Ran and MOG1. Human MOG1 mutants E50K and D70K significantly reduced the binding thereof to Ran.

### BRIEF DESCRIPTION OF DRAWINGS

FIG. 1 is a graph showing results of enhancing myocardial sodium current by an E50K MOG1 mutant and a wild-type MOG1 in HEK293/Nav1.5 cells.
FIG. 2 is a graph showing results of myocardial sodium current density of the E50K MOG1 mutant and the wild-type MOG1 in rat primary cardiomyocytes.
FIG. 3 is a graph showing results of restoring sodium current density of a p.D1275N Nav1.5 mutant by a half dose of the E50K MOG1 super mutant and a full dose of the wild-type MOG1.
FIG. 4 is a graph showing results of restoring sodium current density of a p.G1743R Nav1.5 mutant by a half dose of the E50K MOG1 super mutant and a full dose of the wild-type MOG1.
FIG. 5 is a graph showing results of restoring sodium current density of a p.T353INav1.5 mutant by a half dose of the E50K MOG1 super mutant and a full dose of the wild-type MOG1.
FIG. 6 is a graph showing results of enhancing cell surface expression of Nav1.5 by the E50K MOG1 mutant and the wild-type MOG1 in HEK293/Nav1.5 cells.
FIG. 7 is a graph showing results of binding ability of the E50K MOG1 mutant to Ran.
FIG. 8 is a graph showing a result of the E50K MOG1 mutant reduces an expression level thereof in a cell nucleus, resulting in enhanced expression in cytoplasm.
FIG. 9 is a schematic diagram of the E50K MOG1 mutant of the invention treating arrhythmias and heart failure caused by SCNSA loss-of-function mutations.

### DESCRIPTION OF EMBODIMENTS

In order to make the purpose, technical solutions, and advantages of the invention more clearly understood, the invention is further described in detail below together with the accompanying drawings and embodiments. It should be understood that the specific embodiments described herein are only used to explain the invention, and the embodiments are not used to limit the invention. In addition, the technical features involved in the various embodiments of the invention described below may be combined with each other as long as the features do not conflict with each other.

A mutant of the invention is an E50K mutant, which is the codon GAA encoding glutamic acid (E) at position 50 mutated to the codon AAA encoding lysine (K). The MOG1 gene has four transcripts, and the invention uses the A-type transcript AF265206, in which the encoded MOG1 protein is the longest, with a total of 186 amino acids. The B-type transcript AF265205 encodes MOG1 with a total of 146 amino acids; the third transcript NM_001177802 encodes MOG1 with a total of 165 amino acids; the fourth transcript NM_001330127 encodes MOG1 with a total of 118 amino acids. All transcripts contain glutamic acid at position 50.

### Example 1

The E50K MOG1 mutant enhances myocardial sodium current density more effectively than the wild-type MOG1. MOG1 is a small protein of approximately 22 kD originally discovered to be a Ran-GTP release factor regulating the level of Ran-GTP in the cell nucleus and is involved in protein nuclear transfer. MOG1 is mainly localized in the cell nucleus, and the interaction thereof with Ran is highly conserved in evolution. The mouse MOG1 mutant R30A is hyperactive for GTP release by enhancing the binding to Ran-GTP. Recently, nuclear magnetic resonance (NMR)-based structural analysis combined with biochemical characterization revealed that several salt bridges (RanR95-MOG150, RanR106-MOG1D27, RanK130-MOG1D70, RanK132-MOG153, and RanK134-MOG153) are involved in the interaction between Ran and MOG1. In 2018, Bao et al. found that human MOG1 mutants E50K and D70K significantly reduced the binding thereof to Ran.

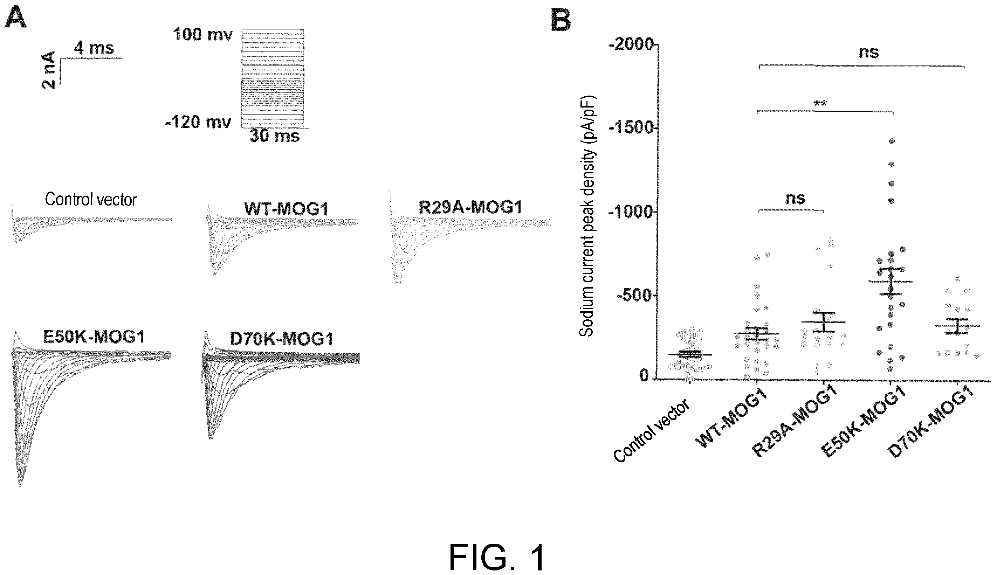

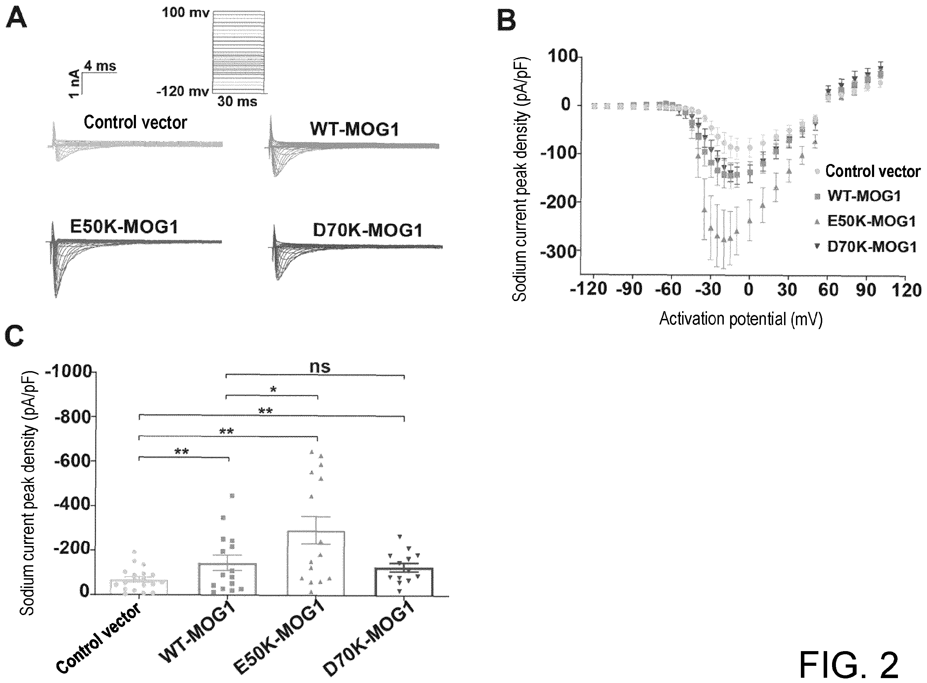

Then the invention established three mutants in human MOG1, respectively R29A, E50K, and D70K, and the effects of the mutants that may affect MOG1-Ran interaction on cardiac sodium ion channel function and cardiac sodium current (INa) density are examined. In the invention, the three mutants, a wild-type MOG1 positive control, and an empty plasmid negative control were transfected into HEK293-Nav1.5 cells respectively, and the changes of sodium current were analyzed and recorded by whole-cell patch clamp. Compared with the control plasmid, all mutants of the invention significantly increased the peak density of cardiac sodium current INa (A in FIG. 1, and B in FIG. 1). In addition, compared with the wild-type (WT) MOG1, the E50K-MOG1 mutant increases by about 2.14 times (-593.9±75.61 pA/pF VS-276.4±34.05, P<0.01, the E50K-MOG1 VS the wild-type MOG1). The results showed that the E50K-MOG1 mutant can increase the peak density of cardiac sodium current INa to a greater extent than the wild type. As shown in FIG. 1, in HEK293/Nav1.5 cells, the E50K MOG1 mutant is more effective than the wild-type MOG1 in enhancing myocardial sodium current. (A) is a schematic diagram of the sodium current. (B) is the peak sodium current density. *, P<0.05, **, P<0.01, ns, not significant (n=15 to 34 cells per group). Therefore, the E50K-MOG1 mutant is more functional than the wild-type MOG1. The same results were verified in rat primary cardiomyocytes (A in FIG. 2, B in FIG. 2, and C in FIG. 2). As shown in FIG. 2, the E50K MOG1 mutant is more effective than the wild-type MOG1 in enhancing myocardial sodium current density in rat primary cardiomyocytes. A is an original schematic diagram of sodium current recorded by whole-cell patch clamp. B is a sodium current I-V curve (current vs voltage; activated from -120 mV to 100 mV). C is peak sodium current density (pA/pF). *, P<0.05, **, P<0.01, ns, not significant (n=13 to 19 cells per group).

### Example 2

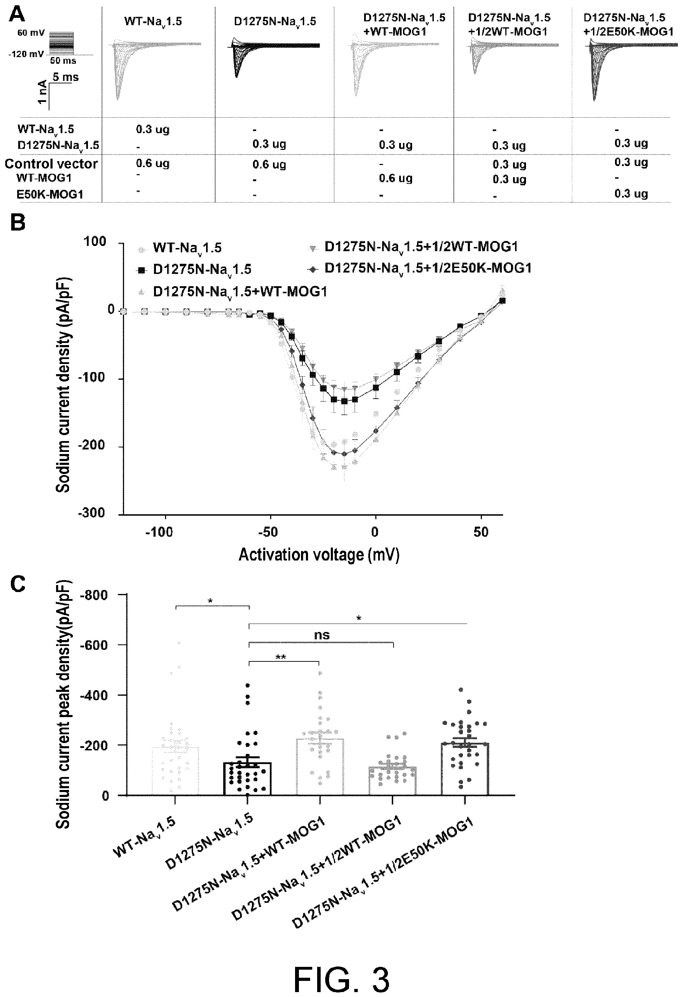

The E50K MOG1 mutant hyperfunctionally restores sodium current abnormality caused by the SCNSA mutation p.D1275N. We transfected wild-type Nav1.5 and the p.D1275N Nav1.5 mutant with different amounts of wild-type MOG1 p3XFLAG-CMV10-WT-MOG1 or mutant MOG1 p3XFLAG-CMV10-E50K-MOG1 into tsA-201 cell lines and then recorded sodium currents by patch clamp. The p.D1275N SCNSA mutant reduced the sodium current peak density by approximately 32.48% (-132.8±19.76 vs -196.7±24.33, D1275N-Nav1.5 vs wild-type Nav1.5). 0.6 µg of the wild-type MOG1 can restore the sodium current density of D1275N-Nav1.5, but 0.3 µg of the wild-type MOG1 cannot enhance the sodium current density of the p.D1275N mutant. On the contrary, 0.3 µg of the E50K MOG1 mutant can successfully restore the sodium current density of D1275N-Nav1.5, with the same potency as 0.6 µg of the wild-type MOG1 (A in FIG. 3, B in FIG. 3, and C in FIG. 3). As shown in FIG. 3, a half dose of the E50K MOG1 super mutant is comparable to a full dose of the wild-type MOG1 in restoring the sodium current density of the p.D1275N Nav1.5 mutant. *, P<0.05, **, P<0.01, ns, not significant (n=25 to 31 cells per group).

### Example 3

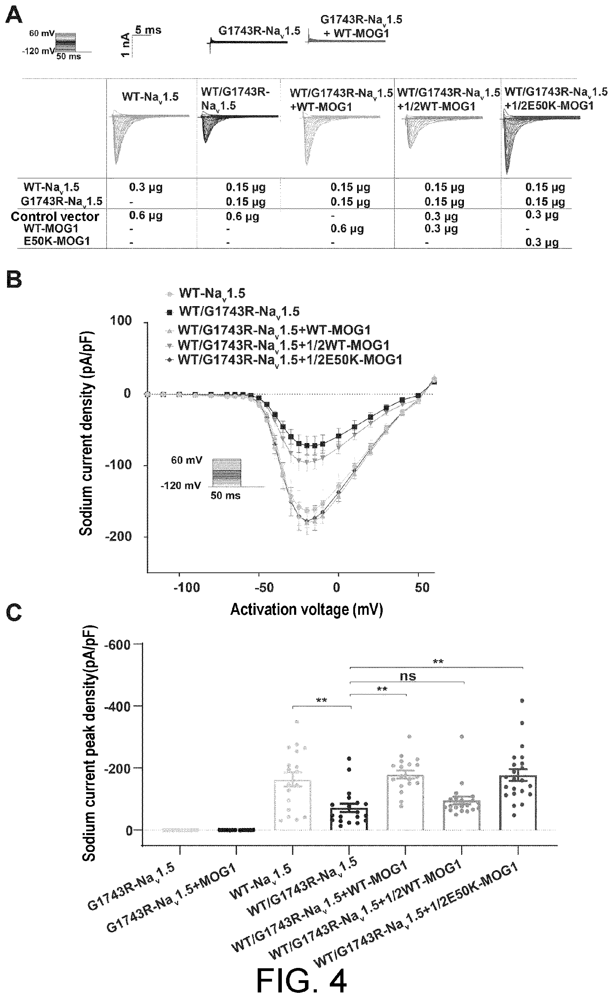

The E50K MOG1 mutant hyperfunctionally restores sodium current abnormality caused by the SCNSA mutation p.G1743R. The same experiment as the p.D1275N Nav1.5 mutant above demonstrated that 0.3 ug of the E50K MOG1 mutant can successfully restore the sodium current density of G1743R-Nav1.5, with the same potency as 0.6 µg of the wild-type MOG1 (A in FIG. 4, B in FIG. 4, and C in FIG. 4). As shown in FIG. 4, a half dose of the E50K MOG1 super mutant is comparable to a full dose of the wild-type MOG1 in restoring the sodium current density of the p.G1743R Nav1.5 mutant. *, P<0.05, **, P<0.01, ns, not significant (n=14 to 22 cells per group).

### Example 4

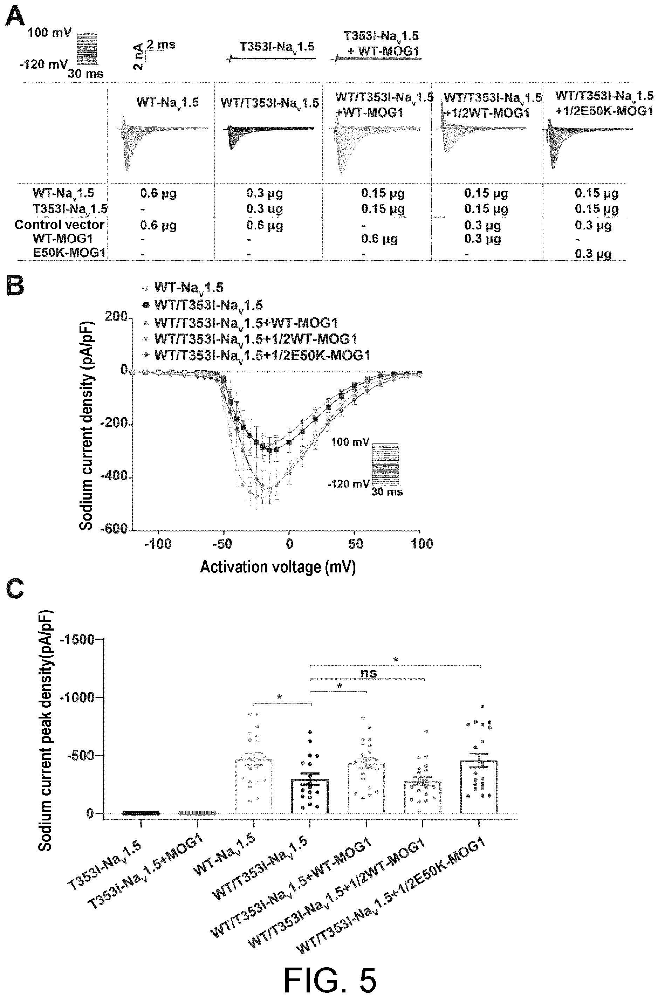

The E50K MOG1 mutant hyperfunctionally restores sodium current abnormality caused by the SCNSA mutation p.T353I. The Nav1.5 mutation p.T353I causes Brugada syndrome. Compared to wild-type channels, the sodium current was reduced by approximately 74%. The same experiments as the p.D1275N and p.G1743R Nav1.5 mutants above demonstrated that 0.3 ug of the E50K MOG1 mutant can successfully restore the sodium current density of T353I-Nav1.5, with the same potency as 0.6 µg of the wild-type MOG1 (A in FIG. 5, B in FIG. 5, and C in FIG. 5). As shown in FIG. 5, a half dose of the E50K MOG1 super mutant is comparable to a full dose of the wild-type MOG1 in restoring the sodium current density of the p.T353I Nav1.5 mutant. *, P<0.05, **, P<0.01, ns, not significant (n=14 to 22 cells per group).

### Example 5

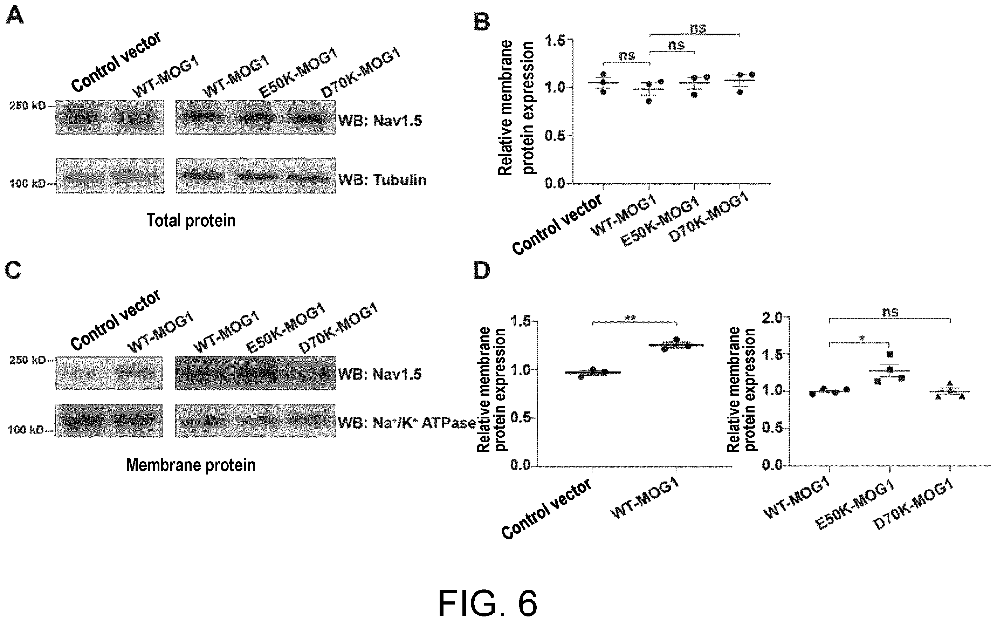

The E50K MOG1 mutant is more effective than the wild-type MOG1 in enhancing cell surface expression of Nav1.5. In HEK293/Nav1.5 cells, similar with the wild-type MOG1, neither the E50K nor D70K MOG1 mutants affected total intracellular Nav1.5 protein expression (A in FIG. 6, and B in FIG. 6). However, for Nav1.5 on the cell membrane surface, the wild-type MOG1 significantly increased the expression level of Nav1.5 on the cell surface compared with the control vector (C in FIG. 6, and D in FIG. 6). Compared with the wild-type MOG1, the E50K MOG1 mutant significantly increased the expression level of Nav1.5 on the cell surface, while the D70K MOG1 mutant had no such effect (C in FIG. 6, and D in FIG. 6). As shown in FIG. 6, in HEK293/Nav1.5 cells, the E50K MOG1 mutant is more effective than the wild-type MOG1 in enhancing the cell surface expression of Nav1.5. A is a schematic diagram of Western blotting (WB) of total Nav.15 expression in cells. B is a quantitative statistical analysis similar to the Western blotting diagram of (A). C is a schematic diagram of Western blotting of Nav.15 expression on the cell membrane surface. D is a quantitative statistical analysis similar to the Western blotting diagram of (C). *, P<0.05, **, P<0.01, ns, not significant. Therefore, the molecular mechanism by which the E50K MOG1 is more effective than the wild-type MOG1 mutant in enhancing the cardiac sodium current is that the E50K MOG1 mutant is more effective than the wild-type MOG1 in transporting Nav1.5 to the cell surface.

### Example 6

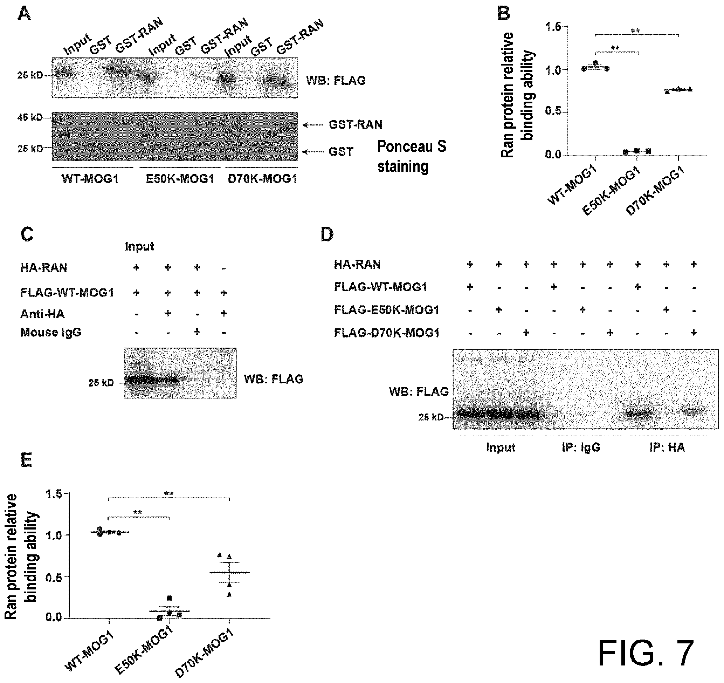

The E50K MOG1 mutant almost lost the binding thereof to Ran. In order to further discover the molecular mechanism by which the E50K MOG1 mutant enhances the sodium current, the invention examined the binding ability of the mutant to Ran through glutathione-S-transferase (GST) pulldown experiment. GST-Ran was used as bait to examine the ability thereof to pull down MOG1. Compared with the wild-type MOG1, the E50K MOG1 mutant reduced the binding to Ran by about 95%, while the D70K MOG1 mutant reduced the binding by only about 26% (A in FIG. 7, and B in FIG. 7). In addition, the invention further verifies the experimental results of GST pulldown by co-immunoprecipitation. In tsA-201 cells, there was an interaction between MOG1 and Ran (C in FIG. 7). Compared with the wild-type MOG1, the E50K MOG1 mutant reduced binding to Ran by approximately 92%, while the D70K MOG1 mutant reduced binding by approximately 47% (D in FIG. 7, and E in FIG. 7). A refers to the GST pull-down experiment. GST-Ran or control GST protein was purified from Escherichia coli by glutathione agarose as bait, and the results are shown by carmine staining (bottom). The wild-type MOG1, E50K, or D70K MOG1 mutants tagged with FLAG were transfected into human tsA201 cells for expression. Cell lysate was used as prey. The bait and the prey were mixed for GST pull-down analysis. Then, the interaction was examined by Western blot using anti-FLAG antibody. B is a quantitative statistical analysis similar to the Western blotting diagram of (A). C refers to the co-immunoprecipitation experiment. The FLAG-tagged MOG1 and HA-tagged Ran were co-expressed in TSA-201 cells, which were then lysed and incubated with HA antibody or control mice IgG, precipitated, then the interaction between the wild-type MOG1 and the wild-type Ran was examined by Western blot using anti-FLAG antibody. D is the co-immunoprecipitation experiment to examine the effect of MOG1 mutants on the interaction with Ran. E is a quantitative statistical analysis similar to the Western blotting diagram of (D). **, P<0.01 (n=3 per group).

### Example 7

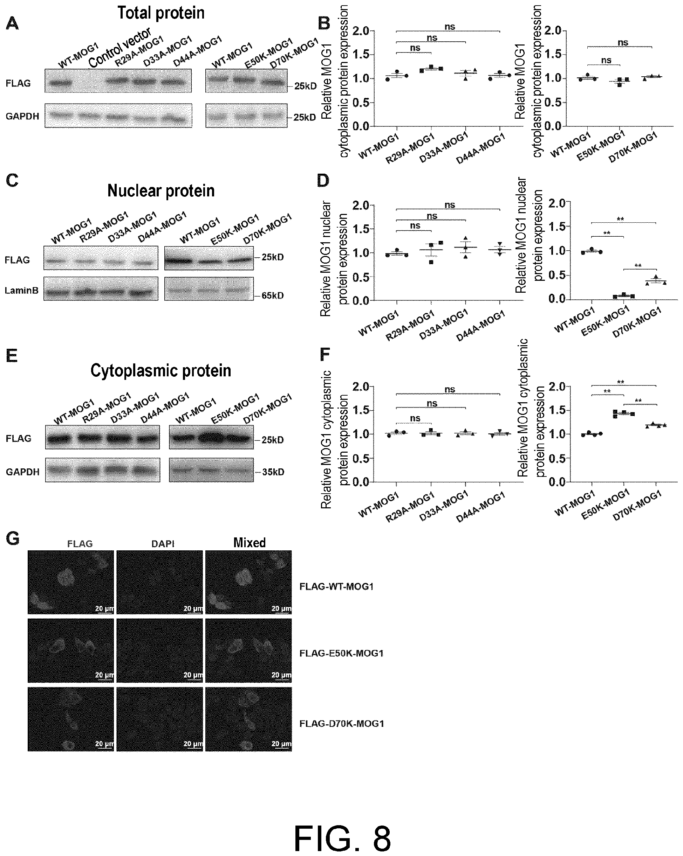

The E50K MOG1 mutant reduces the expression thereof in the cell nucleus, which leads to enhanced expression in the cytoplasm. Since the E50K MOG1 mutant almost completely lost the binding ability to Ran, we believe that the MOG1 mutant can reduce the expression thereof in the cell nucleus, which leads to a highly increased expression in the cytoplasm. We expressed wild-type and mutant MOG1 carrying a FLAG tag in tsA-201 cells and found that the total protein expression levels of wild-type and mutant MOG1 were comparable (A in FIG. 8, and B in FIG. 8). However, after separation of cell nuclear components and cytoplasmic components, it was found that the expression of E50K MOG1 and E70K MOG1 mutants in the cell nucleus was reduced by approximately 95.72% and approximately 63.32% respectively (C in FIG. 8, and D in FIG. 8), and the E50K MOG1 and E70K MOG1 mutants increased the expression thereof in the cytoplasm by approximately 42.67% and approximately 19.14% respectively (E in FIG. 8, and F in FIG. 8). The R29A, D33A, or D44A MOG1 mutants did not affect the expression thereof in the cell nucleus or cytoplasm. We also examined the expression of the E50K MOG1 mutant in HEK293 cells by immunofluorescence. As shown in G in FIG. 8, the wild-type MOG1 is evenly distributed in the cell nucleus and cytoplasm; the D70K MOG1 mutant has reduced expression in the cell nucleus and increased expression in the cytoplasm; the E50K MOG1 mutant was hardly expressed in the cell nucleus, and there expression level thereof was abundant in the cytoplasm. As shown in FIG. 8, the E50K MOG1 mutant reduces the expression level thereof in the cell nucleus, which leads to enhanced expression in the cytoplasm. A is a schematic diagram of Western blotting. FLAG-tagged wild-type and MOG1 mutants were transfected in tsA-201 cells, and the total MOG1 protein expression was examined by anti-FLAG antibody. B is a quantitative statistical analysis similar to the Western blotting diagram of (A). C is a schematic diagram of Western blotting of cell nuclear proteins. D is a quantitative statistical analysis similar to the Western blotting diagram of (C). E is a schematic diagram of Western blotting of cytoplasmic proteins. F is a quantitative statistical analysis similar to the Western blotting diagram of (E). G refers to immunofluorescence staining showing the specific location of MOG1 protein in tsA-201 cells. FLAG-tagged MOG1 plasmid was transfected into tsA-201 cells, MOG1 protein was incubated with anti-FLAG antibody and then incubated with secondary antibody, and cell nucleus was visualized by blue DAPI staining. Scale bar, 20 um.

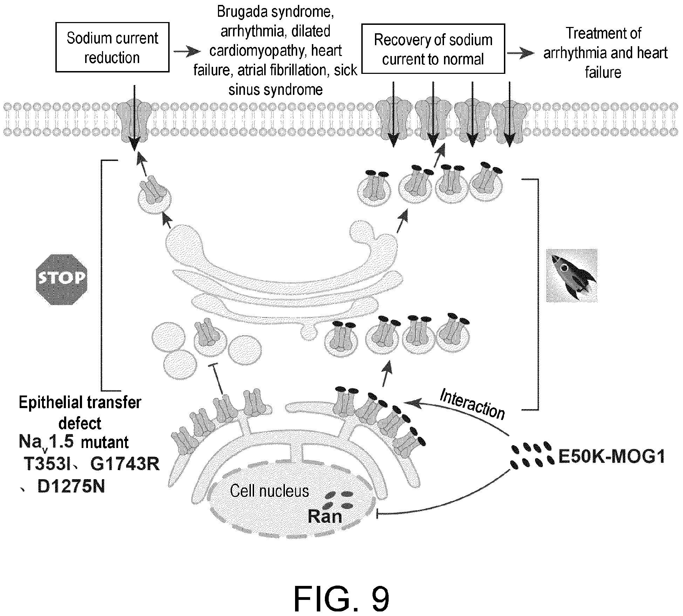

FIG. 9 is a schematic diagram of the E50K MOG1 mutant of the invention treating arrhythmias and heart failure caused by SCNSA loss-of-function mutations. The loss-of-function mutations, such as p.G1743R, p.D1275N, and p.T353I, reduce the sodium current density generated by the cardiac sodium channel Nav1.5, which causes diseases such as Brugada syndrome, dilated cardiomyopathy, heart failure, atrial fibrillation, and sick sinus syndrome. The E50K MOG1 mutant can successfully replenish the effects of the Nav1.5 mutants on cardiac sodium channel function and cardiac sodium current density, and the replenishing intensity thereof is higher than the wild type. The molecular mechanism thereof is that the E50K MOG1 mutant inhibits the interaction between MOG1 and the cell nuclear protein Ran, thereby the number of MOG1 entering the cell nucleus is reduced, which significantly increases the number thereof in the cytoplasm. Therefore, the E50K MOG1 mutant can interact with Nav1.5 more effectively, facilitate the epithelial transfer of Nav1.5, and ultimately replenish the sodium current to normal levels, thereby the purpose of treating the disease is achieved.

It should be easily understood by persons skilled in the art that the above description is only preferred embodiments of the invention and the embodiments are not intended to limit the invention. Any modifications, equivalent substitutions, and improvements made within the spirit and principles of the invention should be included in the protection scope of the invention.

## Claims

1. A mutant of a small molecule chaperone protein MOG1-encoding gene, **characterized in that** a base sequence of the mutant is as shown in SEQ ID NO: 1.

2. A mutant of a small molecule chaperone protein MOG1, **characterized in that** an amino acid sequence of the mutant is shown in SEQ ID NO: 2.

3. A use of the mutant of the small molecule chaperone protein MOG1-encoding gene as claimed in claim 1 for preparing drugs for treating Brugada syndrome, arrhythmia, dilated cardiomyopathy, or heart failure.

4. The use as claimed in claim 3, **characterized in that** the arrhythmia is ventricular tachycardia, ventricular fibrillation, sick sinus syndrome, or atrial fibrillation.

5. The use as claimed in claim 3 or 4, **characterized in that** the mutant of the small molecule chaperone protein MOG1-encoding gene is used to enhance a sodium current density of cardiomyocytes.

6. A use of the mutant of the small molecule chaperone protein MOG1 as claimed in claim 2 for preparing drugs for treating Brugada syndrome, arrhythmia, dilated cardiomyopathy, or heart failure.

7. The use as claimed in claim 6, **characterized in that** the arrhythmia is ventricular tachycardia, ventricular fibrillation, sick sinus syndrome, or atrial fibrillation.

8. The use as claimed in claim 6 or 7, **characterized in that** the mutant of the small molecule chaperone protein MOG1 is used to enhance a sodium current density of cardiomyocytes.
